# EUROPEAN PATENT SPECIFICATION

(11) **EP 2 848 259 B1**
(45) Date of publication and mention of the grant of the patent: **23.03.2016**
(21) Application number: 14184419.1
(22) Date of filing: 11.09.2014
(51) Int. Cl.: A61K 47/44, A61K 9/20, A61K 31/16

(54) **Sustained release pharmaceutical formulations of Thiocolchicoside**
Pharmazeutische Formulierungen mit verzögerter Freisetzung von Thiocolchicosid
Formulations pharmaceutiques à libération prolongée de thiocolchicoside

(30) Priority: 12.09.2013 TR 201310731
(43) Date of publication of application: 18.03.2015
(73) Proprietor: Sanovel Ilac Sanayi ve Ticaret A.S., 34460 Istanbul (TR)
(72) Inventor: Türkyilmaz, Ali, 34460 Istanbul (TR); Pehlivan Akalin, Nur, 34460 Istanbul (TR); Durgun, Deniz Ömür, 34460 Istanbul (TR)
(74) Representative: Sevinç, Erkan

(56) References cited:
- WO-A1-2011/112161
- WO-A1-2012/144964
- WO-A2-2010/103544

## Description

### Technical Aspect

Present invention relates to novel sustained release pharmaceutical formulations containing an effective amount of thiocolchicoside or pharmaceutically acceptable salts thereof for oral administration with myorelaxant activity.

### Background of the Invention

Muscle relaxants are used in the management of musculoskeletal and neuromuscular disorders. They also reduce muscle tone and are used in therapy for the treatment of muscle spasm and contractures.

Muscle spasm is one of the main factors responsible for chronic pain; it characterises several pathologies of the locomotor apparatus as well as inflammatory-rheumatic and degenerative orthopaedic pathologies. When it affects joints, they cause not only pain, but also rigidity, which reduces joint mobility and flexibility in the affected part. Muscle contractures also characterize several pathologies of the locomotor apparatus and are one of the main factors responsible for the persistence of the pain associated to these pathologies.

For these reasons, the study of molecules endowed with muscle relaxant and antispasmodic properties still raises remarkable interest from the clinical point of view. As it is known, colchicine is a pseudoalcaloid that has been widely used for a long time in therapy for the treatment of gout. The use of 3- demethyl- thiocolchicine glucoside, known as thiocolchicoside, is also widespread in therapy for treating contractures and inflammatory conditions that affect the muscular system.

Thiocolchicoside has been claimed to possess GABA-mimetic and glycinergic actions. In other words we can say that thiocolchicoside is a gamma-aminobutiric acid receptor agonist and it is used in the treatment of painful muscle spasms or spasticity occurring in musculoskeletal and neuromuscular disorders and for treating contractures and inflammatory conditions that affect the muscular system. Its chemical structure is shown in Formula 1.

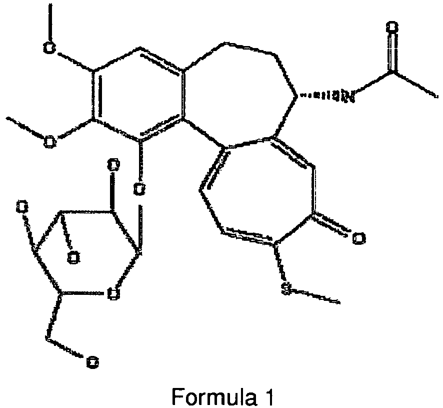

The usual initial dose is 16 mg daily by mouth. It has also been given intramuscularly, in doses up to 8 mg daily, or applied as cream or ointment.

It is well known to those skilled in the art that the blood plasma concentration levels of drugs need to be maintained above a minimum effective level and below its minimum toxic level in order to obtain the desired therapeutic effects and to minimize side effects. It is also known in the prior art that, thiocolchicoside is a highly soluble active ingredient and almost completely absorbed after oral administration. Therefore, thiocolchicoside that is administered in the form of a conventional tablet or a capsule causes plasma concentration level to be initially very high, followed by a rapid decline, and this results in a transient dose dumping, followed by a long period of under dosing. So, it leads to fluctuations of drug concentration in the blood plasma and undergoes repeated chemical imbalances, which might be detrimental to the patient's health. Consequently, it fails to maintain the desired therapeutic effect for a long time and leads to a need to obtain an extended duration of therapeutic effect with minimum potential for side effects as well as to provide high patient compliance that is essential for successful treatment period.

The various formulations of thiocolchicoside are described in prior art as follows:
EP 1052995B1 discloses a pharmaceutical composition containing thiocolchicoside which is in the form of a powder and is administered by nasal administration.
WO1998036751A1 discloses a solid pharmaceutical composition of thiocolchicoside for administration via the buccal mucosa in the absorption site.

WO 1996/041635A1 discloses a pharmaceutical composition containing a diclofenac salt and thiocolchicoside.

Taking consideration of the prior art, there is still a need for a sustained release pharmaceutical formulation of thiocolchicoside which comes over aforementioned disadvantages and providing additional advantages over the art.

Accordingly the object of the invention is to develop a sustained release pharmaceutical formulation of thiocolchicoside that provides once daily or twice daily dosing for effective management of pain, spasticity, inflammatory symptoms and painful muscle spasms, an improved side effect profile and increased patient compliance.

### Objects of the Invention

The object of the present invention is to provide a sustained release pharmaceutical formulation containing an effective amount of thiocolchicoside or pharmaceutically acceptable salts thereof and at least one rate controlling polymer for the treatment of pain, spasticity, inflammatory symptoms and painful muscle spasms by eliminating all the aforementioned drawbacks and providing additional advantages to the respective technical field.

Another object of the present invention is to provide a sustained release pharmaceutical formulation ensuring the release of thiocolchicoside in a controlled manner over an extended period of time to maintain substantially constant drug levels in blood plasma and avoid the fluctuations associated with the conventional immediate release formulations.

Another object of the present invention is to provide a sustained release pharmaceutical formulation comprising thiocolchicoside with desired dissolution rate of said molecule.

Another object of the present invention is to provide a sustained release pharmaceutical formulation maintaining requisite blood levels for an extended time period sufficient to justify once daily or twice daily dosing and thus increase patient compliance.

Another object of the present invention is to provide an sustained release pharmaceutical formulation ensuring the desired sustained release of thiocolchicoside even if it is formulated in monolayer tablet form.

Another object of the present invention is to provide an sustained release pharmaceutical formulation that is also produced with good physical properties.

Another object of the present invention is to provide a sustained release pharmaceutical formulation that ensures repeatable and pH-independent release of thiocolchicoside.

Another object of the present invention is to provide a process for the manufacture of said sustained release pharmaceutical formulation of thiocolchicoside.

### Description of the figures

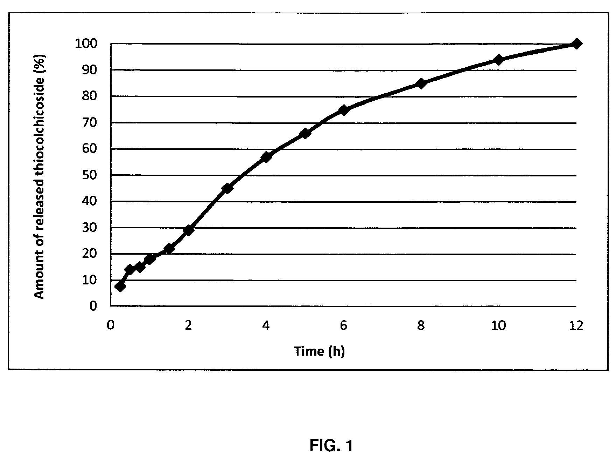

**Figure 1** shows dissolution rate of thiocholchicoside of the sustained release formulation containing glyceryl behenate.

### Detailed description of the Invention

This invention relates to a sustained release pharmaceutical formulation of thiocolchicoside containing at least one rate controlling polymer for oral administration with anti-inflammatory and myorelaxant activity and methods of its manufacture.

The term "sustained release pharmaceutical formulation" refers to any pharmaceutical formulation that maintains constant levels of a drug in the patient's bloodstream by releasing the drug over an extended period of time. Sustained release pharmaceutical formulations are formulated to release the active ingredient gradually and predictably over a 12-hour to 24-hour period.

The term "rate controlling polymer" refers to an excipient in the final dosage form whose primary function is to modify the duration of release of the active drug substance from the dosage form.

The term "dosage form" refers to any form of the formulation that contains the active agent(s) in an amount sufficient to achieve a therapeutic effect with a single administration.

The term "active agent" refers to the agent, ingredient, drug or other substance that provides some beneficial pharmacological effect for the treatment.

The term "pharmaceutically acceptable excipient" refers to any ingredient having no therapeutic activity and being nontoxic and thus suitable as an excipient.

Sustained release pharmaceutical formulations, which are effective in maintaining the drug levels in blood plasma over sustained period of time result in optimal therapy. They reduce the frequency of dosing, as they maintain substantially constant drug levels in blood plasma and avoid the fluctuations associated with the conventional immediate release formulations administered three to four times a day. Three-times daily or four times daily dosing regimens of the conventional immediate release formulations is very inconvenient and leads to reduction in patient compliance. Reduction of dosing regimen from three times daily to twice daily or to once daily results in increased convenience and comfort therefore increased patient compliance.

Additionally, sustained release pharmaceutical formulation for the outpatient provides reduced dosage regimens convenience and, more importantly, better assurance of compliance. For example, the reduction of a dose regimen from four times a day to three times a day allows the patient to take the prescribed drug during waking hours. Reduction of a dose regimen to twice a day allows the patient to take the prescribed drug in the morning and in the evening, which provides greater convenience; e.g., the patient is not required to carry an additional one when away from home. Of course, the most convenient dosage form is a once daily dose regimen. This also reduces the risk of the omitted tablets.

Thiocolchicoside is a highly and rapidly soluble active ingredient and almost completely absorbed after oral administration. It is known that, it is difficult to develop an sustained release pharmaceutical formulations of highly and rapidly soluble pharmaceuticals. It is also hard to achieve the desired dissolution profiles in other words the control of the release rate is difficult. Therefore, fluctuation of the active ingredient concentration in the plasma may occur which may lead to toxicity. Also, diurnal variation of the active ingredient in plasma is also possible.

The present invention provides a novel sustained release pharmaceutical formulations containing an effective amount of thiocolchicoside or pharmaceutically acceptable salts thereof and at least one rate controlling polymer that provides such therapeutic relief by releasing thiocolchicoside in such a manner that dose dumping is prevented and requisite blood levels are maintained for an extended time period sufficient to justify once daily or twice daily dosing and thus increase patient compliance.

Another aspect of the present invention, glyceryl behenate is used as rate controlling polymer in said sustained release pharmaceutical formulation. According to the dissolution studies, formulations of the invention containing glyceryl behenate show an incremental release in more controlled manner for a long time depicting an sustained release pattern (Fig. 1). According to this dissolution studies, 10% to 30% of total amount of thiocolchicoside by weight is released in the first hour in distilled water with stirring by a paddle at the rate of 100 rpm at 37 ºC. This released amount of thiocolchicoside is sufficient to provide minimum effective concentration level in blood plasma for therapeutic effect. Then, sustained release of the remaining thiocolchicoside keeps the plasma concentration efficient for longer duration. Therefore, the desired therapeutic effect is obtained with release of thiocolchicoside in more controlled manner and these results in longer duration effect of thiocolchicoside, providing high patient compliance.

Another of the present invention, the sustained release pharmaceutical formulation of thiocolchicoside is administered orally in tablet, bilayer tablet, multilayer tablet, multicoated tablet, capsule, granulate or pellet form, preferably in tablet form. This tablet form is preferably monolayer tablet form and optionally coated by at least one coating that can contain thiocolchicoside or not.

Additionally, without formulating the sustained release formulation of the invention with another part(s) having thiocolchicoside to form a bilayer or a multi-layer tablet that has some disadvantages (such as requiring additional process steps, being difficult to formulate, etc.) in comparison with monolayer tablets, the desired sustained release profile can be obtained by using glyceryl behenate as rate controlling polymer in said formulation of thiocolchicoside even if it is formulated in monolayer tablet form.

Furthermore, glyceryl behenate has good binding properties , it does not also affect tablet hardness and is unaffected by mixing or production parameters. Therefore, glyceryl behenate makes possible the manufacture of tablets of present invention with the good physical properties in terms of hardness, friability, weight variation, thickness and drug content uniformity, besides showing desired release profile. Additionally, glyceryl behenate, that is also used as a lipophilic matrix agent in formulations, prevents the moisture ingress into the tablets because of its hydrophobic properties and thus, that helps maintaining the stability of the tablet. The hydrophobic properties of glyceryl behenate also enable the use of glyceryl behenate in less amount than hydrophilic substances for controlling the release of the active agent.

Consequently, use of glyceryl behenate as rate controlling polymer enables the sustained release pharmaceutical formulation of thiocolchicoside of the present invention to be more advantageous over the prior art in order to achieve desired therapeutic effects over a longer time period while ensuring high patient compliance and reducing potential side effects.

Another aspect of the present invention, the ratio of total weight of glyceryl behenate to the total weight of thiocolchicoside is between 25:1 to 1:25, preferably between 15:1 to 1:10, more preferably between 10:1 to 1:5.

Another aspect of the present invention, said sustained release pharmaceutical formulation may contain additional rate controlling polymer selected from a group comprising glyceryl palmitostearte, glyceryl monostearate, polyglycolized glycerides, hydrogenated vegetable oils such as hydrogenated castor oil, microcrystalline wax, cetyl alcohol, a polymethacrylate or mixtures thereof.

Another aspect of the invention, the sustained release pharmaceutical formulation further comprise a polymethacrylate, that is also identified as ammonio methacrylate copolymer, as a rate controlling polymer. Although thiocolchicoside is a highly and rapidly soluble active ingredient, its solubility can be affected from different pH values. It is observed that the use of said ammonio methacrylate copolymer with glyceryl behenate in the formulation of the invention enables the sustained release pharmaceutical formulation of the invention ensure repeatable and pH-independent release of thiocolchicoside. Therefore, it is guaranteed that the release rate ensures optimal concentrations of thiocolchicoside in blood plasma in order to achieve effects over a longer period.

According the present invention, ammonio methacrylate copolymer is preferably in aqueous dispersion form with low permeability and this aqueous dispersion contains a polymer dry weight content preferably between 10% and 50%, more preferably between 25% to 50%, most preferably of 30% (available under tradename Eudragit RS 30 D) based on the total weight of the aqueous dispersion.

During production of the sustained release pharmaceutical formulation according to the present invention, firstly, ammonio methacrylate copolymer is added in aqueous dispersion form. Howewer, after drying step of the production, water content of the aqueous dispersion is substantially lost, and the polymer dry weight content is remained in the final sustained release formulation. Therefore, the polymer dry weight content of ammonio methacrylate copolymer remained in the final formulation is between 0.5% to 15%, preferably between 1% to 12%, more preferably between 2% to 9% by weight based on the total weight of the final formulation.

According to the present invention, ammonio methacrylate copolymer aqueous dispersion is preferably used as granulation solution in wet granulation. The percent amount of ammonio methacrylate copolymer aqueous dispersion used is between 1% to 30%, preferably between 5% to 25%, more preferably between 7% to 20% by weight based on the total weight of the formulation.

Another aspect of the present invention, the sustained release pharmaceutical formulation contains thiocolchicoside or pharmaceutically acceptable salts thereof that is present in an amount of between 2 mg and 20 mg, preferably it is present in an amount of between 8 mg and 16 mg.

According to the present invention, sustained release pharmaceutical formulations of the invention can be sterilized and can be in an oral dosage forms include tablets, multilayer tablets (coated or uncoated), multicoated tablets, capsules, hard or soft gelatin capsules, pellets, powders, granules, colloidal dispersions, dispersions, sterile solutions or suspensions and emulsions and the like.

Another aspect of the present invention, the sustained release pharmaceutical formulation further comprises at least one pharmaceutically acceptable excipient selected from a group comprising lubricants, binding agents, fillers, preservatives, disintegrants, plasticizers, aromatic substances, or a mixture thereof. Suitable fillers are selected from the group comprising starch, lactose, microcrystalline cellulose (e.g. Avicel® PH 101), carboxy cellulose sodium, sucrose; suitable binding agents are selected from the group comprising povidone, hydroxypropylcellulose, hydroxypropylmethylcellulose, ethylcellulose, starch, gelatin; suitable lubricants are selected from the group comprising collodial anhydrous silica, magnesium stearate, talc, sodium stearyl fumarate; suitable disintegrants are selected from the group comprising microcrystalline cellulose, sodium starch glycollate, croscarmellose sodium, crospovidone, starch and their mixtures; suitable plasticizer are selected from the group comprising diethyl phthalate (DEP), triethyl citrate; suitable glidants are selected from the group comprising colloidal silicon dioxide, silicon dioxide, magnesium trisilicate, powdered cellulose, starch, talc, tribasic calcium phosphate, lactose, stearates, dibasic calcium phosphate, magnesium carbonate, magnesium oxide, calcium silicate, silicon dioxide aerogels and their mixtures; suitable aromatic agents are selected from the group comprising fruit aromas such as of orange, cherry, strawberry, banana, sourcherry, lemon, etc.; aromas of cardamom, anis, mint, menthol, eucalyptus, vanillin, and ethyl vanillin, and the mixtures thereof; suitable preservatives are selected from the group comprising methylparaben and propylparaben and the salts thereof (e.g. sodium, potassium), sodium benzoate, citric acid, benzoic acid, butylated hydroxytoluene and butylated hydroxyanisole, and the mixtures thereof.

According to the present invention, the sustained release pharmaceutical formulation preferably comprises;
a. 5.0% to 20.0% by weight of thiocolchicoside,
b. 10.0% to 30.0% by weight of glyceryl behenate,
c. 10.0% to 20.0% by weight of ammonio methacrylate copolymer aqueous dispersion,
d. 25.0% to 70.0% by weight of microcrystalline cellulose,
e. 0.5% to 5.0% by weight of talc,
f. 1.0% to 5.0% by weight of sodium stearyl fumarate.

The ammonio methacrylate copolymer aqueous dispersion comprised by the abovementioned formulation contains a polymer dry weight content preferably of 30% (available under tradename Eudragit RS 30 D) based on the total weight of the aqueous dispersion.

According to the invention, the sustained release pharmaceutical formulation of thiocolchicoside is preferably in the form of a tablet, multilayer tablet or multicoated tablet. According to the present invention it may be produced by any standard tabletting technique, e.g. by wet granulation, dry granulation or direct compression.

Preferred process used for the manufacture of the sustained release pharmaceutical formulations of the invention comprising the steps of:
a. thiocolchicoside, glyceryl behenate and microcrystalline cellulose are mixed to form a mixture,
b. obtained mixture is granulated with the ammonio methacrylate copolymer aqueous dispersion,
c. granules are sieved, then dried in a drying oven at the temperature of 40°C-60°C until having moisture content of 2% and milled,
d. talc is mixed with the granules, then sodium stearyl fumarate is added and mixed with granules,
e. optionally, final mixture is pressed to form a tablet.

In wet granulation, it is necessary to obtain stable granules that are not deagglomerated while being dried. In drying step, the wet granules crush each other and they are deagglomerated in small particles. This causes the drug particles to adhere to the filter of the drier and thus an amount of drug particles are remained in the drier. Therefore, obtained doses will be different from each other and it leads to inaccurate dosing. Since it is observed that the granulation solutions lead to above mentioned problem, the granulation solution is selected most carefully to avoid this problem. It is also found that when ammonio methacrylate copolymer aqueous dispersion is used as the granulation solution, the granules stability maintains and the above mentioned problem is not faced. Preferably, the ammonio methacrylate copolymer aqueous dispersion has a polymer dry content preferably between 10% and 50%, more preferably between 25% to 50%, most preferably of 30% based on the total weight of the aqueous dispersion.

The present invention concern the use of the sustained release pharmaceutical formulations comprising thiocolchicoside for the manufacture of a medicament for the treatment of painful muscle spasms associated with static and functional disorders of vertebra or occurred in post-operations of osteoarthritis, pain and inflammatory symptoms associated with tissue trauma, degenerative vertebra diseases as torticollis, dorsalgy, lombalgy, disk hernia, neurologic and traumatic disorders associated with spasticity.

This invention is further defined by reference to the following example(s). Although the examples are not intended to limit the scope of the present invention, it should be considered in the light of the description detailed above.

### Example

This invention comprising thiocolchicoside wherein thiocolchicoside is present in an amount of between 2 mg and 25 mg, preferred embodiments of thiocolchicoside is present in an amount of between 4 mg and 20 mg.

**Table 1: Sustained release pharmaceutical formulation**

| **Content** | **% amount (w/w)** |
|---|---|
| Thiocolchicoside | 8.0 - 15.0 |
| Glyceryl behenate | 15.0 - 27.5 |
| Ammonio methacrylate copolymer aqueous dispersion* | 10.0 - 17.5 |
| Microcrystalline cellulose | 40.0 - 60.0 |
| Talc | 1.0 - 2.5 |
| Sodium stearyl fumarate | 1.0 - 4.0 |

| | |
|---|---|
| **with a polymer dry content of 30% based on the total weight of the aqueous dispersion (Eudragit RS 30 D)* | |

The sustained release pharmaceutical formulation mentioned above is in the form of a tablet having the thiocolchicoside granules. It is prepared as follows: thiocolchicoside, glyceryl behenate and microcrystalline cellulose are mixed to form a mixture; obtained mixture is granulated with ammonio methacrylate copolymer aqueous dispersion with a polymer dry weight content of 30% (under the tradename Eudragit RS 30 D); granules are sieved, then dried in a drying oven at the temperature of 40°C-60°C until having moisture content of 2% and milled until having a particle size of 700 µm; talc is added and mixed with the granules for 5 minutes, then sodium stearyl fumarate is added and mixed with granules for 3 minutes; obtained granules of thiocolchicoside are compressed by tablet press machine to obtain tablet and these tablets are preferably covered by a coating material including conventional coating polymers like Opadry®.

Tablets are tested for 12 hours using the paddle method at 100 rpm using 900 mL of distilled water at 37 ºC. Dissolution test results as follows:

**Table 2: Dissolution profile**

| Time* | 0 m | 15 m | 30 m | 45 m | 60 m | 1.5 h | 2 h | 3 h | 4 h | 5 h | 6 h | 8 h | 10 h | 12 h |
|---|---|---|---|---|---|---|---|---|---|---|---|---|---|---|
| Dissolution, % | 0 | 7.5 | 14 | 15 | 18 | 22 | 29 | 45 | 57 | 66 | 75 | 85 | 94 | 100 |

| | | | | | | | | | | | | | | |
|---|---|---|---|---|---|---|---|---|---|---|---|---|---|---|
| ** m: minute, h:hour* | | | | | | | | | | | | | | |

Dissolution profile is shown in Figure 1.

## Claims

1. A sustained release pharmaceutical formulation comprising an effective amount of thiocolchicoside or pharmaceutically acceptable salts thereof and glyceryl behenate as a rate controlling polymer.

2. The sustained release pharmaceutical formulation according to claim 1, wherein the ratio of total weight of glyceryl behenate to the total weight of thiocolchicoside is between 25:1 to 1:25.

3. The sustained release pharmaceutical formulation according to claim 2, wherein the ratio of total weight of glyceryl behenate to the total weight of thiocolchicoside is between 15:1 to 1:10.

4. The sustained release pharmaceutical formulation according to claim 3, wherein the ratio of total weight of glyceryl behenate to the total weight of thiocolchicoside is between 10:1 to 1:5.

5. The sustained release pharmaceutical formulation according to any of preceding claims further comprising additional rate controlling polymer selected from a group comprising glyceryl palmitostearte, glyceryl monostearate, polyglycolized glycerides, hydrogenated vegetable oils such as hydrogenated castor oil, microcrystalline wax, cetyl alcohol, a polymethacrylate or mixtures thereof.

6. The sustained release pharmaceutical formulation according to claim 5, wherein the additional rate controlling polymer is a polymethacrylate.

7. The sustained release pharmaceutical formulation according to claim 6, wherein polymethacrylate is ammonio methacrylate copolymer.

8. The sustained release pharmaceutical formulation according to claim 7, wherein ammonio methacrylate copolymer is in aqueous dispersion form.

9. The sustained release pharmaceutical formulation according to claim 8, wherein ammonio methacrylate copolymer aqueous dispersion contains a polymer dry weight content between 10% and 50% based on the total weight of the aqueous dispersion.

10. The sustained release pharmaceutical formulation according to claim 9, wherein the polymer dry weight content between 25% to 50% based on the total weight of the aqueous dispersion.

11. The sustained release pharmaceutical formulation according to claim 10, wherein the polymer dry weight content of 30% based on the total weight of the aqueous dispersion

12. The sustained release pharmaceutical formulation according to any of preceding claims, wherein the amount of ammonio methacrylate copolymer aqueous dispersion is between 1% to 30% by weight based on the total weight of the formulation.

13. The sustained release pharmaceutical formulation according to any of preceding claims, wherein said pharmaceutical formulation is in the form of a tablet, bilayer tablet, multilayer tablet, multicoated tablet, capsule, granulate or pellet.

14. The sustained release pharmaceutical formulation according to any of preceding claims comprises:
a. 5.0% to 20.0% by weight of thiocolchicoside,
b. 10.0% to 30.0% by weight of glyceryl behenate,
c. 10.0% to 20.0% by weight of ammonio methacrylate copolymer aqueous dispersion,
d. 25.0% to 70.0% by weight of microcrystalline cellulose,
e. 0.5% to 5.0% by weight of talc,
f. 1.0% to 5.0% by weight of sodium stearyl fumarate.

15. A process for the manufacture of the sustained release pharmaceutical formulation according to any of preceding claims comprising the steps of:
a. thiocolchicoside, glyceryl behenate and microcrystalline cellulose are mixed to form a mixture,
b. obtained mixture is granulated with the ammonio methacrylate copolymer aqueous dispersion,
c. granules are sieved, then dried in a drying oven at the temperature of 40°C-60°C until having moisture content of 2% and milled,
d. talc is mixed with the granules, then sodium stearyl fumarate is added and mixed with granules,
e. optionally, final mixture is pressed to form a tablet.

## Patentansprüche

1. Pharmazeutische Formulierung mit nachhaltiger Freisetzung, aufweisend eine wirksame Menge von Thiocolchicosid oder pharmazeutisch annehmbarer Salze davon und Glycerylbehenat als ein ratenkontrollierendes Polymer.

2. Pharmazeutische Formulierung mit nachhaltiger Freisetzung nach Anspruch 1,
wobei das Verhältnis des Gesamtgewichts von Glycerylbehenat zu dem Gesamtgewicht von Thiocolchicosid zwischen 25:1 bis 1:25 ist.

3. Pharmazeutische Formulierung mit nachhaltiger Freisetzung nach Anspruch 2,
wobei das Verhältnis des Gesamtgewichts von Glycerylbehenat zu dem Gesamtgewicht von Thiocolchicosid zwischen 15:1 bis 1:10 ist.

4. Pharmazeutische Formulierung mit nachhaltiger Freisetzung nach Anspruch 3,
wobei das Verhältnis des Gesamtgewichts von Glycerylbehenat zu dem Gesamtgewicht von Thiocolchicosid zwischen 10:1 bis 1:5 ist.

5. Pharmazeutische Formulierung mit nachhaltiger Freisetzung nach einem der voranstehenden Ansprüche, weiter aufweisend zusätzliches ratenkontrollierendes Polymer, welches aus einer Gruppe, aufweisend Glycerylpalmitostearat, Glycerylmonostearat, polyglycolysierte Glyceride, hydrierte Pflanzenöle, wie zum Beispiel hydriertes Rizinusöl, microcrystallines Wachs, Cetylalkohol, ein Polymethacrylat oder Mischungen hiervon, ausgewählt ist.

6. Pharmazeutische Formulierung mit nachhaltiger Freisetzung nach Anspruch 5,
wobei das weitere ratenkontrollierende Polymer ein Polymethacrylat ist.

7. Pharmazeutische Formulierung mit nachhaltiger Freisetzung nach Anspruch 6,
wobei das Polymethacrylat ein Ammoniummethacrylatcopolymer ist.

8. Pharmazeutische Formulierung mit nachhaltiger Freisetzung nach Anspruch 7,
wobei das Ammoniummethacrylatcopolymer in Form einer wässrigen Dispersion ist.

9. Pharmazeutische Formulierung mit nachhaltiger Freisetzung nach Anspruch 8,
wobei die wässrige Ammoniummethacrylatcopolymer-Dispersion einen Gewichtsanteil an trockenem Polymer zwischen 10% und 50% basierend auf dem Gesamtgewicht der wässrigen Dispersion enthält.

10. Pharmazeutische Formulierung mit nachhaltiger Freisetzung nach Anspruch 9,
wobei der Gewichtsanteil an trockenem Polymer zwischen 25% bis 50% basierend auf dem Gesamtgewicht der wässrigen Dispersion ist.

11. Pharmazeutische Formulierung mit nachhaltiger Freisetzung nach Anspruch 10,
wobei der Gewichtsanteil an trockenem Polymer 30% basierend auf dem Gesamtgewicht der wässrigen Dispersion ist.

12. Pharmazeutische Formulierung mit nachhaltiger Freisetzung nach einem der voranstehenden Ansprüche,
wobei die Menge an wässriger Ammoniummethacrylatcopolymer-Dispersion zwischen 1% bis 30% basierend auf dem Gesamtgewicht der Formulierung ist.

13. Pharmazeutische Formulierung mit nachhaltiger Freisetzung nach einem der voranstehenden Ansprüche,
wobei die pharmazeutische Formulierung in Form einer Tablette, einer zweischichtigen Tablette, einer mehrschichtigen Tablette, einer mehrschichtig beschichteten Tablette, einer Kapsel, eines Granulats oder von Pellets ist.

14. Pharmazeutische Formulierung mit nachhaltiger Freisetzung nach einem der voranstehenden Ansprüche aufweisend:
a. 5,0% bis 20,0% nach Gewicht an Thiocolchicosid,
b. 10,0% bis 30,0% nach Gewicht an Glycerylbehenat,
c. 10,0% bis 20,0% nach Gewicht an wässriger Ammoniummethacrylatcopolymer-Dispersion,
d. 25,0% bis 70,0% nach Gewicht an microcrystalliner Cellulose,
e. 0,5% bis 5,0% nach Gewicht an Talk,
f. 1,0% bis 5,0% nach Gewicht an Natriumstearylfumarat.

15. Verfahren zum Herstellen einer pharmazeutischen Formulierung mit nachhaltiger Freisetzung nach einem der voranstehenden Ansprüche, die Schritte aufweisend:
a. Thiocolchicosid, Glycerylbehenat und microcrystalline Cellulose werden gemischt, um eine Mischung zu bilden,
b. die erhaltene Mischung wird mit wässriger Ammoniummethacrylatcopolymer-Dispersion granuliert,
c. die Granulate werden gesiebt, dann in einem Trocknungsofen bei einer Temperatur von 40°C - 60°C getrocknet, bis sie einen Feuchtigkeitsanteil von 2% haben, und gemahlen,
d. Talk wird mit den Granulaten gemischt, das Natriumstearylfumarat hinzugegeben, und mit den Granulaten gemischt,
e. wahlweise wird die Mischung abschließend in Form von Tabletten gepresst.

## Revendications

1. Une formulation pharmaceutique à libération prolongée comprenant une quantité efficace de thiocolchicoside ou de l'un de ses sels pharmaceutiquement acceptalbes et du béhénate de glycéryle en tant que polymère de contrôle de vitesse de libération.

2. La formulation pharmaceutique à libération prolongée selon la revendication 1, dans laquelle le rapport du poids total de béhénate de glycéryle sur le poids total de thiocolchicoside est compris entre 25 :1 et 1 :25.

3. La formulation pharmaceutique à libération prolongée selon la revendication 2, dans laquelle le rapport du poids total de béhénate de glycéryle sur le poids total de thicolchicoside est compris entre 15 :1 et 1 :10.

4. La formulation pharmaceutique à libération prolongée selon la revendication 3, dans laquelle le rapport du poids total de béhénate de glycéryle sur le poids total de thiocolchicoside est compris entre 10 :1 et 1 :5.

5. La formulation pharmaceutique à libération prolongée selon l'une quelconque des revendications précédentes comprenant en outre un polymère supplémentaire de contrôle de vitesse de libération choisi dans le groupe comprenant le palmitostéarate de glycéryle, de monostéarate de glycéryle, les glycérides polyglycolisés, les huiles végétales hydrogénées telles que l'huile de ricin hydrogénée, une cire microcristalline, l'alcool cétylique, un polyméthacrylate ou des mélanges de ces composés.

6. La formulation pharmaceutique à libération prolongée selon la revendication 5, dans laquelle le polymère supplémentaire de contrôle de vitesse de libération est un polyméthacrylate.

7. La formulation pharmaceutique à libération prolongée selon la revendication 6, dans laquelle le polyméthacrylate est un copolymère de méthacrylate d'ammonium.

8. La formulation pharmaceutique à libération prolongée selon la revendication 7, dans laquelle le copolymère de méthacrylate d'ammonium est sous forme de dispersion aqueuse.

9. La formulation pharmaceutique à libération prolongée selon la revendication 8, dans laquelle la dispersion aqueuse de copolymère de méthacrylate d'ammonium contient une teneur sèche en polymère comprise entre 10 et 50% en poids rapporté au poids total de la dispersion aqueuse.

10. La formulation pharmaceutique à libération prolongée selon la revendication 9, dans laquelle la teneur sèche en polymère est comprise entre 25 et 50% en poids rapporté au poids total de la dispersion aqueuse.

11. La formulation pharmaceutique à libération prolongée selon la revendication 10, dans laquelle la teneur sèche en polymère est de 30% en poids rapporté au poids total de la dispersion aqueuse.

12. La formulation pharmaceutique à libération prolongée selon l'une quelconque des revendications précédentes, dans laquelle la quantité de dispersion aqueuse de copolymère de méthacrylate d'ammonium est comprise en 1 et 30% en poids rapporté au poids total de la formulation.

13. La formulation pharmaceutique à libération prolongée selon l'une quelconque des revendications précédentes, dans laquelle ladite formulation pharmaceutique est sous la forme d'un comprimé, d'un comprimé bicouche, d'un comprimé multicouche, d'une gélule, d'un granulé ou d'un pellet.

14. La formulation pharmaceutique à libération prolongée selon l'une quelconque des revendications précédentes comprenant :
a. 5,0 à 20,0% en poids de thiocolchicoside,
b. 10,0 à 30,0% en poids de béhénate de glycéryle,
c. 10,0 à 20,0% en poids de dispersion aqueuse de copolymère de méthacrylate d'ammonium,
d. 25,0 à 70,0% en poids de cellulose microcristalline,
e. 0,5 à 5,0% en poids de talc,
f. 1,0 à 5,0% en poids de fumarate de stéaryl-sodium.

15. Un procédé de fabrication d'une formulation pharmaceutique à libération prolongée selon l'une quelconque des revendications précédentes, comprenant les étapes suivantes :
a. du thicolchicoside, du béhénate de glycéryle et de la cellulose microcristalline sont mélangés pour former un mélange,
b. le mélange obtenu est granulé avec la dispersion aqueuse de copolymère de méthacrylate d'ammonium,
c. les granulés sont tamisés, puis séchés dans une étuve à une température de 40 à 60°C jusqu'à ce que leur teneur en humidité soit de 2% et broyés,
d. du talc est mélangé avec les granulés, ensuite du fumarate de stéaryl-sodium est ajouté et mélangé avec les granulés,
e. éventuellement, le mélange final est pressé pour former un comprimé.
